# EUROPEAN PATENT SPECIFICATION

(11) **EP 1 144 000 B1**
(45) Date of publication and mention of the grant of the patent: **25.01.2006**
(21) Application number: 00901367.3
(22) Date of filing: 11.01.2000
(51) Int. Cl.: A61K 38/55, A61M 1/14, A61P 7/02

(54) **NEW DIALYSIS METHOD**
NEUE DIALYSEMETHODE
NOUVEAU PROCEDE DE DIALYSE

(30) Priority: 11.01.1999 SE 9900043
(43) Date of publication of application: 17.10.2001
(73) Proprietor: AstraZeneca AB, 151 85 Södertälje (SE)
(72) Inventor: FAGER, Gunnar, S-431 83 Mölndal (SE)
(86) International application number: PCT/SE2000/000030
(87) International publication number: WO 2000/041715

(56) References cited:
- EP-A1- 0 884 325
- WO-A1-93/11152
- WO-A1-94/29336
- WO-A1-97/30073
- WO-A1-98/48800
- WO-A1-98/48804
- WO-A1-98/50420

## Description

### Field of the Invention

This invention relates to a new use of thrombin inhibitors, particularly low molecular weight thrombin inhibitors.

### Introduction

Haemodialysis is a process for removing waste products and toxins from the blood of patients with renal malfunction or failure. Blood is removed from, and returned to, circulation, either through an artificial arteriovenous fistula or a temporary or permanent internal catheter, and passes through an "artificial kidney", or dialyser.

Dialysers vary in design and performance, but all include a dialysis membrane and a dialysing solution. This solution may contain essential electrolyte salts and buffer(s) including sodium chloride, potassium chloride, magnesium chloride, calcium chloride and acetic acid. The concentrations are carefully selected (sometimes individually for each patient) with the aim of restoring normality to electrolyte imbalances.

Dialysing solutions, which may also contain glucose, sodium bicarbonate, lactic acid and EDTA, may be prepared by carefully regulated dilution of a concentrated stock (a dialysis concentrate) using sterile, pyrogen-free water, or may be provided in ready-to-use form.

In dialysis, toxins are removed by diffusion through the dialysis membrane, thus restoring blood to its normal state. However, the process has to be repeated at regular intervals (e.g. two to three times a week for four to six hours a session).

In the haemodialysis of patients with chronic renal failure, the natural blood clotting process, which may take place on dialysis membranes and in blood lines, gives rise to significant problems including inefficient filtration and/or premature termination of the dialysis session.

The most widely used currently-available methodology for the prevention of this problem involves the pre-administration of a parenteral bolus dose of anticoagulant. The anticoagulant compound may be heparin, which may be used in unfractionated (UH; MW approximately 5,000 to 30,000), or low molecular weight (LMWH; MW around 4,000), forms. Intravenous administration of bolus heparin typically takes place before dialysis is conducted.

Although pre-dosing with heparin may alleviate problems such as those mentioned above, it is by no means an entirely satisfactory solution, and indeed further complications are often observed. For example, the effective dose of the heparin that may be used with individual patients has to be pre-determined by titration, to avoid over- or under-dosing, which may give rise to bleeding, and clotting on dialysis membranes and in blood lines, respectively. The wrong dose may thus give rise to severe bleeding or premature termination of the dialysis session. Moreover, the compound may escape via the dialysis filter and the anticoagulant effect thereof decrease during the dialysis process, leading to thrombotic occlusions of dialysis fistulas or catheters. Furthermore, heparin-induced thrombocytopenia is known to occur in as much as 3% of patients with chronic renal failure, and heparin-induced osteoperosis may also occur in some patients. Low molecular weight heparins are also expensive (up to ten times more expensive than ordinary heparin).

Thus, there is a need for an alternative approach, which may provide for a safer, more reliable and more efficacious anticoagulant effect during dialysis, and especially haemodialysis.

### Prior Art

International patent application WO 94/29336 discloses, generically and specifically, compounds that are useful as thrombin inhibitors, and thereby as anticoagulants. The thrombin inhibiting compounds that are specifically mentioned include HOOC-CH₂-(*R*)Cgl-Aze-Pab-H, which is also known as melagatran (see WO 94/29336 and the list of abbreviations therein). Haemodialysis is mentioned as one of the many indications, for which the disclosed compounds are stated to be useful.

French patent application FR 2 687 070 discloses dialysis concentrates comprising *inter alia* sodium heparinate. The use of low molecular weight thrombin inhibitors is not mentioned.

### Disclosure of the Invention

We have found, surprisingly, that the above-mentioned problems may be solved by adding a low molecular weight thrombin inhibitor to the dialysing solution, prior to, and/or during, dialysis, such as haemodialysis.

According to a first aspect of the invention there is provided the use of a low molecular weight thrombin inhibitor in the manufacture of a medicament for the treatment by dialysis, particularly by haemodialysis, of a patient in need of such treatment, in which the thrombin inhibitor is provided in the dialysing solution.

By treatment of patients "in need of treatment by dialysis", we include the therapeutic and/or prophylactic treatment of (i.e. providing a therapeutic and/or prophylactic anticoagulant effect (which may be at least in part extracorporeal) during dialysis in) patients with, for example, renal complications, including the therapeutic and/or prophylactic treatment of patients with diseases that may lead to renal complications and/or renal failure, including chronic and/or acute renal failure. The term also includes the therapeutic and/or prophylactic treatment of patients with intoxication by compounds that may give rise to organ damage, severe metabolic disturbances and/or death.

We have found, in particular, and surprisingly, that when low molecular weight thrombin inhibitors are provided in the dialysing solution, they pass freely through dialysis membranes, and may thus provide highly predictable, reproducible and stable concentrations of anticoagulant on the patient side of the membrane, and thereby in the patient, throughout the entire dialysis session.

Low molecular weight thrombin inhibitors may thus be provided as part of a dialysing solution ready for use in dialysis (i.e. by dissolving, or dispersing, the inhibitor in the dialysing solution, ready for use in the dialyser). However, we prefer that low molecular weight thrombin inhibitors are provided as part of a dialysis concentrate, which concentrate is to be diluted by an appropriate means before being used as part of a dialysing solution.

We have found, advantageously, that low molecular weight thrombin inhibitors may be used with standard dialysis, e.g. haemodialysis, concentrates. "Standard" dialysis concentrates that may be mentioned include any currently (e.g. commercially) available concentrate known to those skilled in the art, but will also include any pharmaceutical composition which may be used as a dialysis concentrate, i.e. which comprises components that endow the properties necessary to enable such use. The skilled person will appreciate that properties that are necessary to enable a formulation's use as a dialysis concentrate include properties that enable the formation, upon dilution with e.g. sterile, pyrogen-free water, of an appropriate dialysing solution, which resultant solution may have an osmolarity of between 270 and 300, preferably 280 and 295 mOsm/L, and should enable the provision of molar gradients of ions between that solution and blood, such that the dominating mass transport of endogenously accumulated ions is to the dialysing solution, and the dominating mass transport of endogenously deprived ions is in the opposite direction. The resultant dialysing solution may therefore include between 135 and 142 mmol/L of Na⁺ ions, between 0 and 2 mmol/L of K⁺ ions, between 1.25 and 2 mmol/L of Ca²⁺ ions, between 0.5 and 1 mmol/L of Mg²⁺ ions, between 107 and 115 mmol/L of Cl⁻ ions, and may also contain between 2 and 35 mmol/L of acetate ions, between 0 and 38 mmol/L of HCO₃⁻ ions, between 0 and 6 mmol/L of glucose, as well as EDTA and lactate. The skilled person will appreciate that suitable concentrates may also be acid and/or alkaline-buffered, and may comprise other ingredients, which ingredients may be used to endow the resultant solution with the properties mentioned hereinbefore, and/or which may allow the avoidance of problems that may be associated with renal failure, including accumulation of fluid.

Dialysis concentrates including low molecular weight thrombin inhibitors may thus be prepared by admixing a thrombin inhibitor, or a formulation including such an inhibitor, with other components of a dialysis concentrate, in accordance with techniques which are known to the skilled person. The resultant concentrate may be employed in standard dialysers in accordance with known techniques.

Dialysis concentrate including low molecular weight thrombin inhibitor may be provided for use in dialysis in a form that contains thrombin inhibitor, or may alternatively be provided as a kit of parts for use in dialysis comprising (a) a formulation including a low molecular weight thrombin inhibitor, and (b) a dialysis concentrate.

Low molecular weight thrombin inhibitors may be provided for use in such a kit of parts in a formulation that may be readily admixed with a dialysis concentrate, for example as the inhibitor itself (e.g. in solid form), or pre-dissolved or pre-dispersed in a pharmaceutically acceptable carrier that may be admixed with the concentrate in order to obtain a concentrate in which the inhibitor is dissolved or evenly dispersed.

Suitable dialysis concentrates for use in the kit of parts preferably include those mentioned hereinbefore, but may also include an amount of the same, or a different, thrombin inhibitor as/to that used in the other component of the kit of parts.

The term "low molecular weight thrombin inhibitor" will be understood by those skilled in the art. The term may also be understood to include any composition of matter (e.g. chemical compound) which inhibits thrombin to an experimentally determinable degree in *in vivo* and/or in *in vitro* tests, and which possesses a molecular weight of below 2,000, preferably below 1,000, or, in the context of this invention, a prodrug of such a composition/compound.

Preferred low molecular weight thrombin inhibitors include low molecular weight peptide-, amino acid-, and/or peptide analogue-based thrombin inhibitors.

The term "low molecular weight peptide-, amino acid-, and/or peptide analogue-based thrombin inhibitors" will be well understood by one skilled in the art to include thrombin inhibitors, and, in the context of this invention, prodrugs of thrombin inhibitors, with one to four peptide linkages, and/or with a molecular weight below 1000, and includes those compounds (active thrombin inhibitors and prodrugs of active thrombin inhibitors, as appropriate) described in the review paper by Claesson in Blood Coagul. Fibrin. (1994) **5,** 411, as well as those disclosed in US Patent N° 4,346,078; International Patent Applications WO 93/11152, WO 95/23609, WO 93/05069, WO 97/46577, WO 98/01422, WO 95/35309, WO 96/25426, WO 94/29336, WO 93/18060, WO 95/01168, WO 97/23499, WO 97/02284, WO 97/46577, WO 96/32110, WO 98/06740, WO 97/49404, WO 98/57932, WO 99/29664, WO 96/31504, WO 97/11693, WO 97/24135 and WO 97/47299; and European Patent Applications 648 780, 468 231, 559 046, 641 779, 185 390, 526 877, 542 525, 195 212, 362 002, 364 344, 530 167, 293 881, 686 642, 669 317, 601 459, 623 596, 796 271 and 809 651, the disclosures in all of which documents are hereby incorporated by reference.

Preferred low molecular weight peptide-based thrombin inhibitors include those known collectively as the "gatrans". Particular gatrans which may be mentioned include HOOC-CH₂-(*R*)Cha-Pic-Nag-H (known as inogatran; see International Patent Application WO 93/11152 and the list of abbreviations therein) and HOOC-CH₂-(*R*)Cgl-Aze-Pab-H (known as melagatran; see International Patent Application WO 94/29336 and the list of abbreviations therein) and, in the context of this invention, prodrugs of melagatran (see e.g. WO 97/23499). Particularly preferred thrombin inhibitors include melagatran.

Thrombin inhibitor, or dialysis concentrate comprising thrombin inhibitor, may be provided to the dialysing solution (in the case of concentrate, following dilution with an appropriate quantity of water (e.g. sterile, pyrogen-free water)) in an appropriate amount, that will allow for delivery of the drug to the patient at a controlled rate across the dialysis membrane over the whole dialysis session. This may involve constant infusion to the inlet tubing of the dialysing solution.

Suitable concentrations of low molecular weight thrombin inhibitors in the dialysis concentrate and/or the dialysing solution will depend upon the thrombin inhibitor (and/or prodrug of that inhibitor) which is used, the severity of the disorder to be treated and the nature of the patient to be treated, but can be determined non-inventively. Suitable concentrations of low molecular weight thrombin inhibitors and prodrugs that may be used include those which give a mean plasma concentration of thrombin inhibitor that is in the range 0.001 to 100 µmol/L, preferably, 0.005 to 20 µmol/L and particularly 0.009 to 15 µmol/L, once equilibrium is reached, over the period for which treatment is required. Suitable doses for inogatran and prodrugs thereof are those which give a mean plasma concentration in the range 0.1 to 10 µmol/L, and preferably 0.5 to 2 µmol/L; suitable doses for melagatran and prodrugs thereof are those which give a mean plasma concentration in the range 0.01 to 5 µmol/L, and preferably 0.1 to 1 µmol/L.

Maximum plasma concentrations of low molecular weight thrombin inhibitors may be readily determined by the concentration of drug in the dialysing solution, and/or the dialysis concentrate, that is employed. The time taken to reach steady-state equilibrium (at which point the concentrations in the blood and dialysing solution are the same, and at which time passage across the dialysis membrane occurs at equal speeds in both directions) will depend upon factors including the properties of the dialysis membrane that is used, the flow levels in the dialyser, and the physical and chemical properties of the thrombin inhibitor that is employed.

Depending on the severity of the disorder in the patient that is to be treated by dialysis, it may be preferable to administer a bolus dose of thrombin inhibitor initially (by which we include up to 60 minutes prior to the start of the dialysis session), with a view to mitigating thrombotic occlusions of dialysis filters or blood lines that may occur at an early stage during the dialysis session. However, such a treatment is not essential for the performance of the use according to the invention.

The invention described herein may have the advantage that coagulation is reduced in patients in need of dialysis, e.g. haemodialysis, in a manner that is safer, more reliable, more reproducible, more cost-effective and more efficacious than currently available anticoagulation techniques for use in the dialysis, and may thus solve problems associated with these techniques. The present invention may also provide these advantages not only during, but also between, dialyses.

### Examples

The invention is illustrated, but in no way limited, by way of the following examples, in which:
Figure 1 is a schematic representation of a haemodialysis simulation experiment (open single pass system), in which A, B and C are sampling points.
Figure 2 shows a plot of the concentrations of the low molecular weight thrombin inhibitor, melagatran, at inlet C (squares, solid line), outlet B on the Donor side (filled circles, hatched line), and outlet A on the Recipient side (open circles, broken line), against time. The lines are fitted exponential functions.
Figure 3 is a schematic representation of a haemodialysis simulation experiment (closed system of re-circulation), in which A, B and C are sampling points.
Figure 4 shows a plot of the concentrations of the low molecular weight thrombin inhibitor, melagatran, at inlet C (squares, solid line), outlet B on the Donor side (filled circles, hatched line), and inlet A on the Recipient side (open circles, broken line), against time. The lines are fitted exponential functions.
Figure 5 shows a schematic drawing of a haemodialysis set-up in humans, from which the pig study of Example 3 was derived.
Figure 6 shows a plot of the clearance of iohexol in plasma (upper panel), and in the dialysing solution (lower panel), against time during the dialysis session (pig study).
Figure 7 shows a plot of the concentration of melagatran in efferent arterial pig blood and in the dialysis fluid outlet during the dialysis session (pig study).
Figure 8 shows a plot of TAS-ECT times during the dialysis session (pig study), showing the degree of melagatran-induced thrombin inhibition.
Figure 9 shows a plot of APTT times during the dialysis session (pig study), showing the degree of melagatran-induced thrombin inhibition.

### Example 1

### Filtration of Melagatran in an Open Single Pass System - Dialysis Simulation

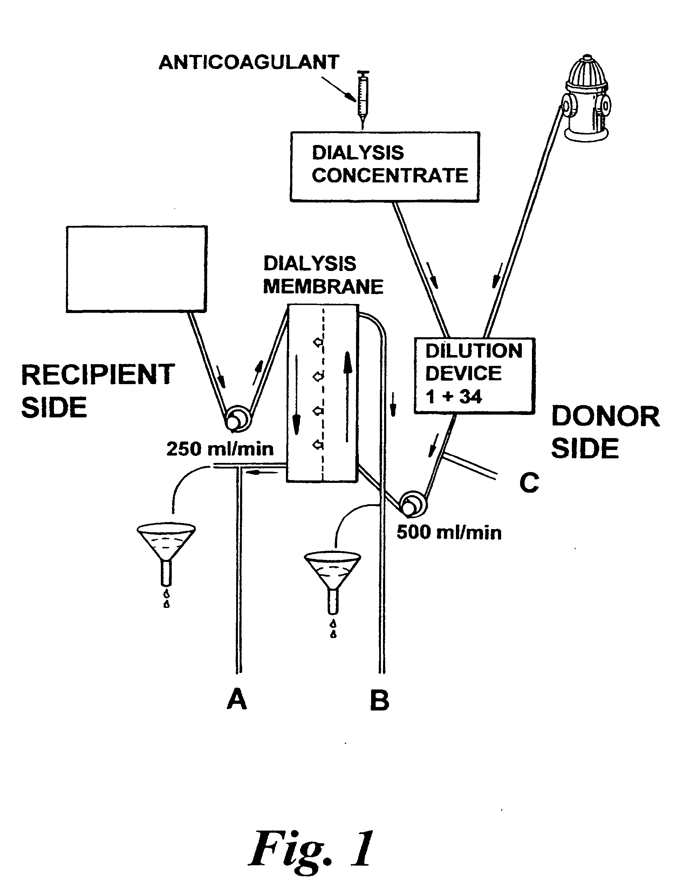

A LunDia Pro 600 (Gambro, Lund, Sweden) dialysis filter was connected to Gambro AK-100 (Gambro) dialysis equipment and primed for 15 minutes with dialysis fluid prepared from Biosol A201.5 glucose (Pharmalink, Solna, Sweden) concentrate (dilution 1:35), The set up was as shown in Figure 1. The dialysis fluid was passed on one side of the membrane (here designated as the Donor side) at a flow of 500 mL/min. Simultaneously, a solution with 0.15 mol/L sodium chloride was pumped through the patient side (here designated as the Recipient side) of the membrane at 250 mL/min and discarded without re-circulation. The flows on the two sides of the membrane were anti-parallel.

The pumps were then stopped and the dialysis concentrate was replaced by a new bag of concentrate containing 3 mg/L (7 µM) of melagatran to provide a final concentration of about 0.2 µM after a 1:35 dilution. The pumps were restarted at the same speeds, except that the dialysis fluid was shunted *via* a collateral circuit past the flter unit. Filter perfusion was resumed at time zero and samples were collected from the tubing at position A (outlet on Recipient side), position B (outlet on Donor side) and position C (inlet on Donor side) at pre-determined time-points over 5 minutes.

The samples (≈ 5 mL) were analysed for the presence of melagatran using a liquid chromatography/mass spectrometry method (Bioanalytical method BA-216). Values below the limit of quantification (10 nM) were set to zero.

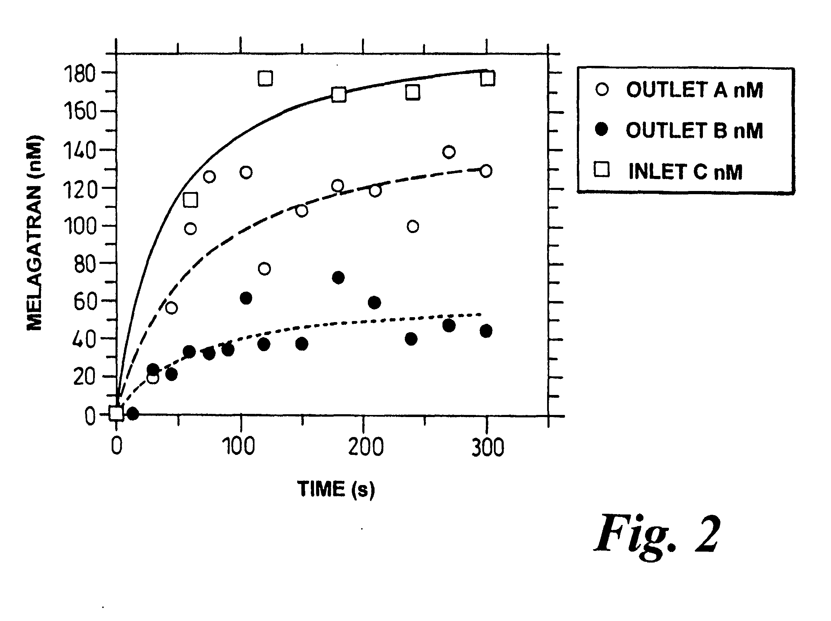

An experimental steady state was reached after about 5 minutes, when a concentration of melagatran of about 180 nM was achieved at the inlet C on the Donor side (see Figure 2). Simultaneously, the concentration of melagatran at the outlet B on the Donor side levelled off at about 50 nM. This suggested that the concentration of melagatran fell by about 130 nM on the Donor side.

On the Recipient side, the concentration of melagatran increased to plateau at about 130 nM after 5 minutes and this was comparable to the decrease on the Donor side. These findings were compatible with anti-parallel flows on the two sides of the membrane; fresh water constantly entering the Recipient side of the membrane during this experiment was first exposed to the low concentration of melagatran in the outlet end on the Donor side and then to increasing concentrations towards the inlet. Consequently, the diffusion gradient across the membrane was about 50 nM and on both sides the gradient along the length of the membrane was 130 nM; it fell from 180 to 50 nM on the donor side and increased from 130 to 0 nM on the recipient side due to the anti-parallel flows.

### Example 2

### Filtration of Melagatran in a Closed Svstem of Re-circulation - Dialysis Simulation

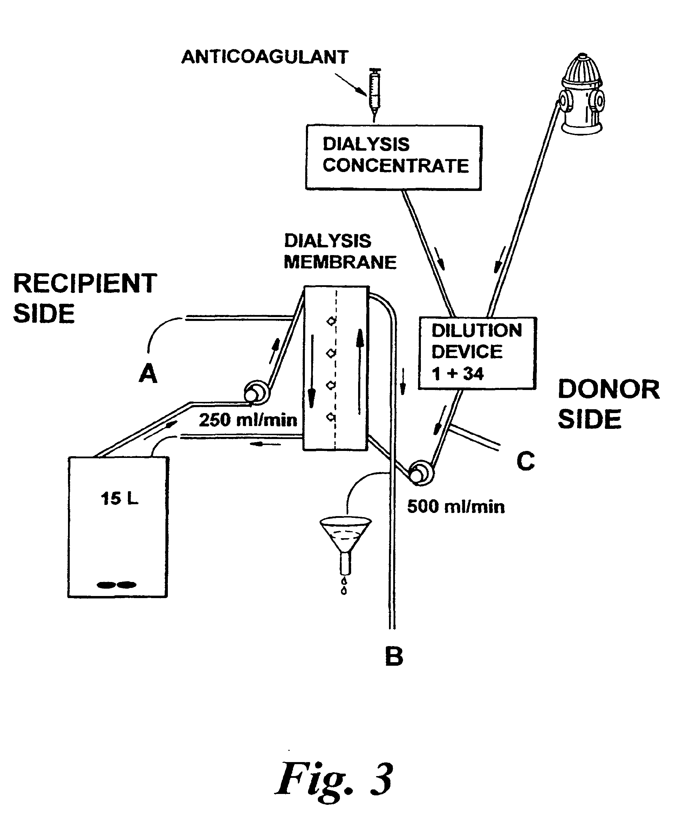

An experiment was carried out in a similar fashion to that described in Example 1, except that the system was closed on the Recipient side to allow re-circulation of fluid *via* a tank containing 15 L of saline and a magnetic stirrer (see Figure 3). The experiment lasted for 120 minutes and samples were collected at pre-determined time-points. Sampling positions B and C were the same as in the previous experiment. However, in this case, the sampling position A was at the filter inlet on the Recipient side down-stream from the tank.

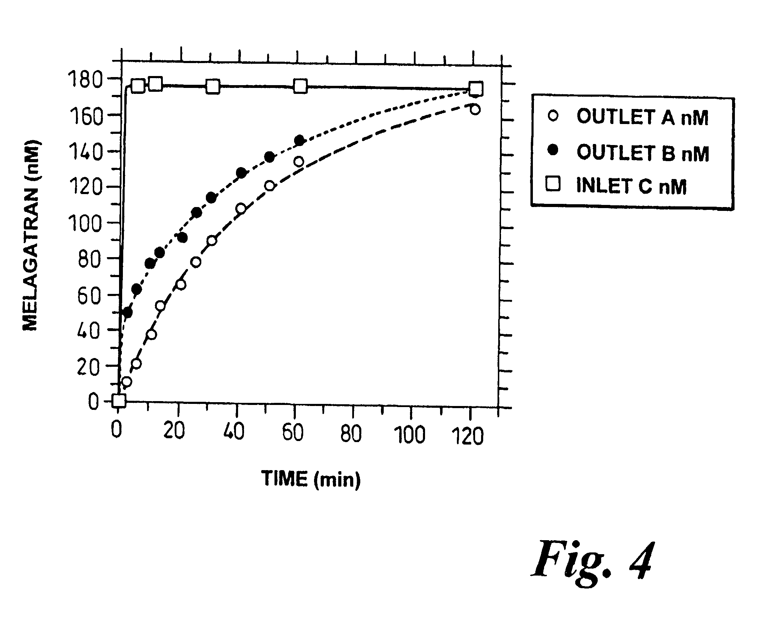

As in Example 1, the concentration of melagatran in the dialysis fluid inlet C on the Donor side reached a plateau at about 180 nM within 5 minutes and remained constant throughout the experiment (see Figure 4).

The gradient between the inlet C and outlet B on the Donor side was about 130 nM when concentrations on the recipient side were small. As melagatran accumulated in the tank, this gradient decreased exponentially. After about 2 hours, the system had equilibrated and this gradient had disappeared; the donor side in- and outlets showed similar concentrations of melagatran of around 180 nM. Simultaneously, the concentration of melagatran in the tank increased and levelled off at about 180 nM with some delay compared to the Donor side. This indicated that melagatran passes through dialysis membranes.

### Example 3

### Pig Study

The aim of this study was to test whether melagatran, given *via* the dialysing solution during dialysis could prevent coagulation and allow a maintained filter function throughout the session in acute experiments with anaesthetised pigs without kidney function.

To avoid very early thrombotic occlusion of blood lines and filters, which, if observed, would have prevented the drawing of meaningful conclusions regarding the utility of the method, an i.v. bolus dose of melagatran was given immediately before the start of extracorporeal blood circulation in these experiments.

### Materials and Methods

Two Swedish Land Race pigs weighing 59 and 57 kg respectively were used. Anaesthesia was induced using Ketaminol® (10 mg/kg, i.m. Veterinaria AG, Switzerland) and Dormicum® (1 to 2 mg/kg, i.m. Roche, Basel, Switzerland), followed by, after 20 minutes, Diprivan® (80 to 160 mg/kg i.v. Zeneca Limited, Macclesfield, Cheshire, United Kingdom), intubated and ventilated at 15 cycles/minute with air containing 2 to 3% Forene® (Abbott Scandinavia AB, Kista, Sweden) using a Servo Ventilator 900C (Siemens Elema, Solna, Sweden).

Blood gases and blood pH were monitored (ABL™ system 625, Radiometer, Copenhagen, Denmark) and adjusted to normal ranges (pH 7.38 to 7.48; pCO₂ 10 to 12 kPa; pO₂ 4.5 to 5.8 kPa) by changes in tidal volume. Ringer solution (Pharmacia & Upjohn AB, Stockholm, Sweden) was infused into the ear vein at 20 mL/kg/h or more, to compensate for fluid losses. Temperature was maintained at 39°C by external heating. ECG was monitored using needle electrodes corresponding to the V3 and V5 positions.

The anaesthetised pigs were subjected to bilateral ligation of renal arteries and veins using lateral flank approaches. The wounds were then closed and the animals were placed on their backs. Two catheters (Kimal, K41/3B/LL, Uxbridge, England) were inserted into the right femoral artery and vein for connection to the dialysis equipment. The artery catheter was used to provide blood to (efferent blood line), and the vein catheter to receive blood from (afferent blood line), the dialysis filter. One polyethylene catheter (Intramedic PE-200, Clay Adams, Parsippany, NJ, USA) was inserted into an artery (right saphenous in pig A and brachial in pig B) for blood pressure (MAP) recording (transducer Peter von Berg Medizintechnik GmbH, Kirchseeon/Eglharting, Germany) and for blood samples, pH and blood gas measurements.

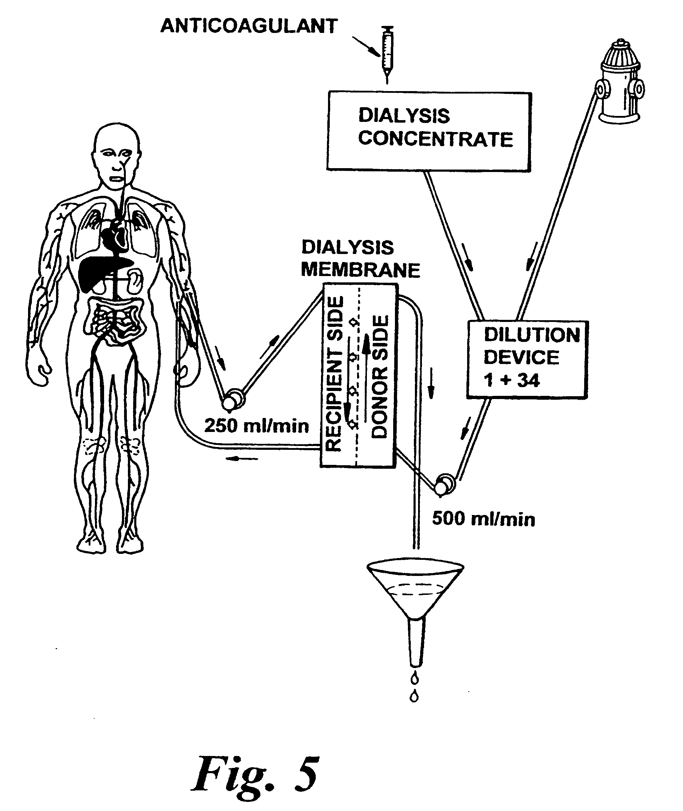

Before the start of dialysis, the LunDia Pro 600 dialysis filter (Gambro, Lund, Sweden) was primed on both sides for 15 minutes with Biosol A201.5 glucose (Pharmalink, Solna, Sweden) dialysis concentrate diluted 1:35 using a Gambro AK-100 (Grambro, Lund, Sweden) haemodialysis equipment (see Figure 5). The dialysis concentrate was supplemented with melagatran (35 µM; thus providing 1 µM after dilution). The flow rate was adjusted to 500 mL/min on the dialysing solution side and to 250 mL/min on the blood side of the membrane by separate pumps. Pressure was measured (transducer Peter von Berg Medizintechnik) on both sides of the dialysis membrane.

Before connection of the efferent and afferent blood lines, 20 mL (300 mg/mL) of iohexol (Omnipaque®, Nycomed AB, Lidingö, Sweden) was administered in the ear vein to monitor filter clearance. Two minutes before the start of dialysis, 0.15 µmol/kg of melagatran was given *via* the right femoral vein as a bolus.

After connection of blood lines and two minutes after the administration of the melagatran bolus dose, dialysis was started and carried out for 3 hours. Then, the procedure was interrupted, the blood lines disconnected and the filter and blood lines washed by pumping saline instead of blood to check for the presence of macroscopic blood clots.

Haemodynamic variables were monitored on a 7 D Grass polygraph (Grass Instruments, Quincy, MA, USA) and sampled on a custom-made system Pharm-Lab 5.0 (AstraZeneca R&D, Mölndal, Sweden).

For the determination of activated partial thromboplastin time (APTT), ecarin clotting time (ECT) and the amount of melagatran in plasma, twelve aliquots of blood were drawn at specific time points into plastic tubes containing one volume of 0.13 M sodium citrate. Plasma was recovered after immediate centrifugation (10,000 g for 5 minutes) and stored at -20°C prior to analysis. For the determination of the amount of iohexol in plasma, blood was drawn in heparinized tubes, centrifuged (10,000 g for 5 minutes) and stored at -20°C prior to analysis.

The amount of melagatran in plasma was quantified using liquid chromatographic and mass spectrometric methods (BA-285, AstraZeneca R&D, Mölndal, Sweden).

ECT was determined in 30 µL citrate plasma using a TAS device (Thrombolytic Assessment System, Cardiovascular Diagnostics Inc., Raleigh, NC) and appropriate solid reagent test cards, as recommended.

APTT was determined using a KC10 A micro coagulometer (Amelung, Lemago, Germany). 25 µL of citrate plasma was incubated with 25 µL of PTT-Automate reagent (Diagnostica Stago, Asnières, France) for 3 minutes. Coagulation was started using 25 µL of 0.025 M CaCl₂ (Diagnostica Stago, Asnières, France) and the time taken to the start of coagulation was measured.

Amounts of iohexol in plasma and dialysing solution were determined at the Laboratory of the Division of Nephrology (Sahlgren's University Hospital, Gothenberg, Sweden) using a Reanalyzer PRX 90 (Provalid AB Lund, Sweden).

### Results

In both pigs, the 3 hour dialysis procedure was carried out successfully. In one pig, the pressure in the efferent blood-line was stable at about 275 mm Hg and, in the other, a small increase from about 250 to 375 mm Hg was observed. After dialysis, filters and tubing were washed clear of blood with saline over 15 minutes, indicating that macroscopic thrombi had not been formed during the procedure.

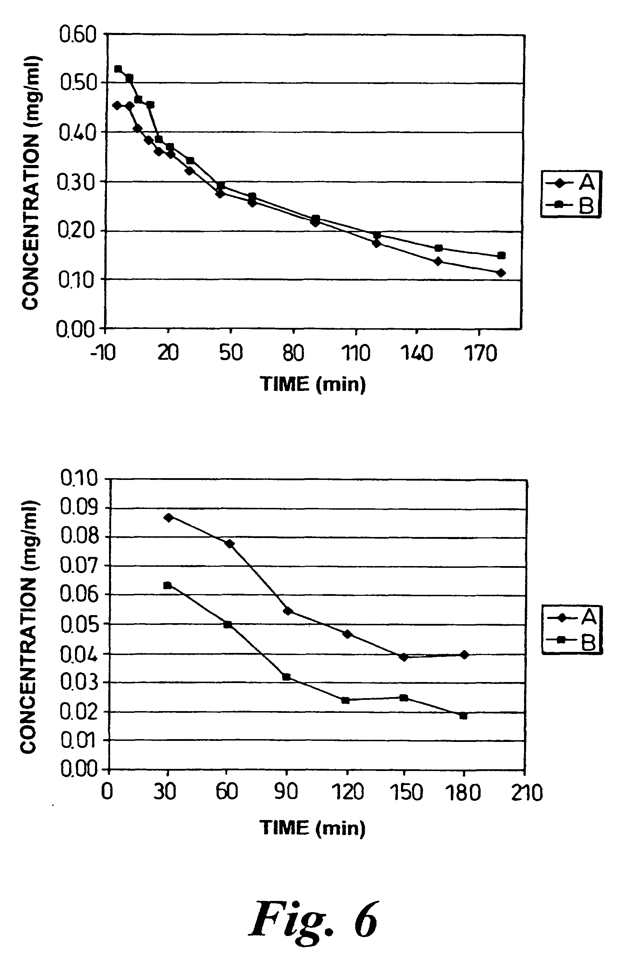

Clearance of iohexol was measured repeatedly every half-hour during the dialysis session to monitor any change in filtration (see Figure 6). The clearance of iohexol was about 150 mL/min in the two experiments. The amount of iohexol in the dialysing solution was proportional to the concentration in plasma at the start of each half-hour period throughout the dialysis session (r² = 0.925, p<0.001 and r² = 0.952, p < 0.003, respectively). This indicated that filter function was maintained over the full 3 hours of dialysis.

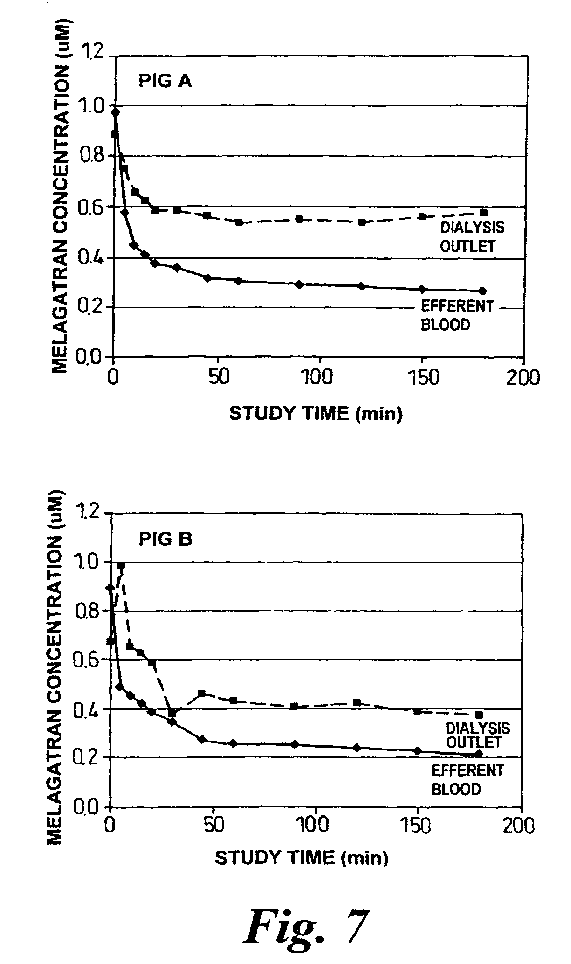

At the start of the dialysis procedure, 2 minutes after an i.v. bolus dose of melagatran of 0.15 M/kg was administered, the concentration of melagatran in plasma from the pigs (efferent blood) was about 0.9 to 1 µM in both pigs (see Figure 7). This declined rapidly, and leveled off at about 0.25 µM over the first 30 minutes of dialysis. After this, plasma concentrations were quite constant. In the dialysis outlet, the concentrations of melagatran were about 0.2 µM higher than on the blood side, except for the very first sample taken at the start of dialysis immediately after the i.v. bolus dose. The concentrations of melagatran in the dialysis concentrate before dilution were 33.7 and 34.9 µM, respectively. Hence, the concentration in the dialysis outlet was clearly less than the 1 µM expected in the dialysis inlet. This indicates that equilibration occurred within one hour in the acutely anuric pig under these experimental conditions.

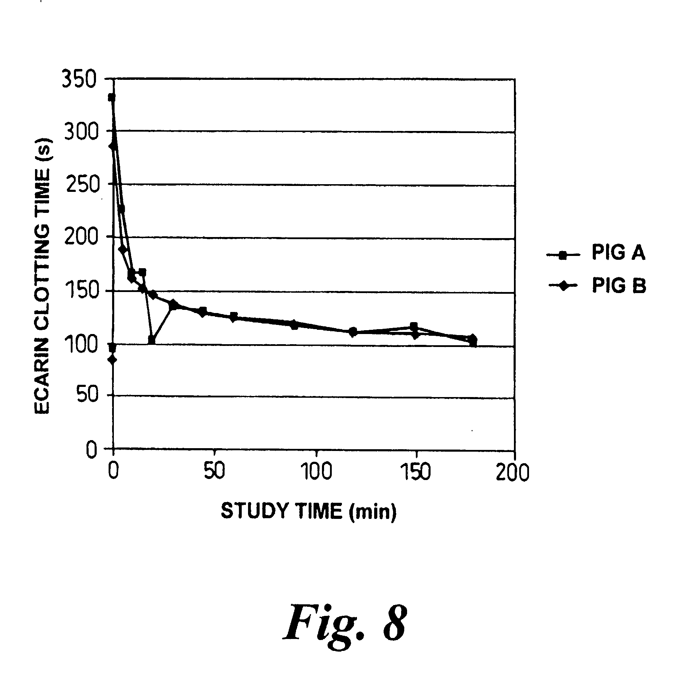

As in previous studies (carried out in normal humans given melagatran s.c.), bedside ecarin clotting times (ECT) using the TAS equipment (see Figure 8) gave a pharmacodynamic measure of melagatran-induced thrombin inhibition, which closely reflected drug concentrations in plasma.

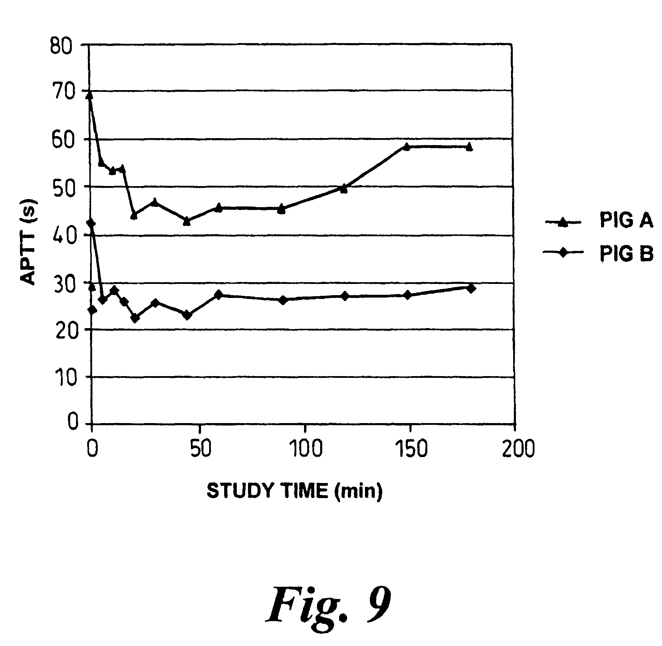

In contrast to the linear relationship between ECT times and melagatran concentrations, APTT times were proportional to the logarithm of melagatran concentration. This was reflected in a larger relative difference in APTT values between the two pigs (see Figure 9).

### Conclusions

Melagatran is useful in preventing extracorporeal clot formation during haemodialysis. Stable antithrombotic levels of melagatran can be obtained during the whole dialysis session by providing the drug in suitable concentrations in the dialysis solution. This demonstrates the usefulness of this new approach. Clearly, administration of a low molecular weight thrombin inhibitor such as melagatran *via* the dialysing solution provides useful plasma concentrations during the whole dialysis session, prevents extracorporeal clot formation and maintains optimal filter function throughout the session.

### Example 4

### Patient Study

An open cross-over study of three different concentrations of melagatran in the dialysis concentrate is carried out. Treatments are given in randomised order to six patients undergoing chronic haemodialysis treatment due to renal insufficiency. Subcutaneous LMWH in the ordinary dose for each patient is given during one session for comparison. The concentrations of melagatran in the dialysis concentrate are selected such that the plasma concentrations of the patient at steady state are about 0.2, 0.3 and 0.4 µmol/l. The results from the pig study of Example 3 are used in the final decision about useful concentrations.

The study provides preliminary data on the feasibility of administering melagatran to haemodialysis patients by filtration from the dialysis fluid, and in the prevention of clotting of dialysis filters. It may also provide preliminary data on useful concentrations of melagatran and a comparison with established LMWH therapy.

## Claims

1. The use of a low molecular weight thrombin inhibitor for the manufacture of a medicament for the treatment by dialysis of a patient in need of such treatment, in which the thrombin inhibitor is provided in the dialysing solution.

2. The use as claimed in Claim 1, wherein the dialysis is haemodialysis.

3. A use as claimed in any one of Claims 1 or 2, wherein the thrombin inhibitor is a low molecular weight peptide-based thrombin inhibitor, a low molecular weight amino acid-based thrombin inhibitor, and/or a low molecular weight peptide analogue-based thrombin inhibitor, or a prodrug of any of these.

4. A use as claimed in Claim 3, wherein the thrombin inhibitor is inogatran or melagatran, or a prodrug thereof.

## Revendications

1. Utilisation d'un inhibiteur de la thrombine de faible poids moléculaire pour la fabrication d'un médicament pour le traitement par dialyse d'un patient ayant besoin d'un tel traitement, dans laquelle l'inhibiteur de la thrombine est fourni dans la solution de dialyse.

2. Utilisation selon la revendication 1, dans laquelle la dialyse est l'hémodialyse.

3. Utilisation selon l'une quelconque des revendications 1 ou 2, dans laquelle l'inhibiteur de la thrombine est un inhibiteur de la thrombine à base de peptides de faible poids moléculaire, un inhibiteur de la thrombine à base d'acides aminés de faible poids moléculaire et/ou un inhibiteur de la thrombine à base d'analogues de peptides de faible poids moléculaire, ou un promédicament de l'un quelconque de ceux-ci.

4. Utilisation selon la revendication 3, dans laquelle l'inhibiteur de la thrombine est l'inogatran ou le mélagatran ou un promédicament de ceux-ci.

## Patentansprüche

1. Verwendung eines niedermolekularen Thrombininhibitors zur Herstellung eines Medikaments für die Dialysebehandlung eines Patienten, der einer derartigen Behandlung bedarf, wobei der Thrombininhibitor in der Dialyselösung bereitgestellt wird.

2. Verwendung nach Anspruch 1, wobei es sich bei der Dialyse um Hämodialyse handelt.

3. Verwendung nach einem der Ansprüche 1 oder 2, wobei der Thrombininhibitor ein niedermolekularer Thrombininhibitor auf Peptidbasis, ein niedermolekularer Thrombininhibitor auf Aminosäurebasis und/oder ein niedermolekularer Thrombininhibitor auf Basis eines Peptidanalogons oder ein Prodrug von einem von diesen ist.

4. Verwendung nach Anspruch 3, wobei der Thrombininhibitor Inogatran oder Melagatran oder ein Prodrug davon ist.
